(19) Europäisches Patentamt
European Patent Office
Office européen des brevets

(11) EP 0 708 113 B1

(12) EUROPÄISCHE PATENTSCHRIFT

(45) Veröffentlichungstag und Bekanntmachung des Hinweises auf die Patenterteilung:
10.02.1999 Patentblatt 1999/06

(51) Int Cl.6: C08B 11/20

(21) Anmeldenummer: 95114763.6

(22) Anmeldetag: 20.09.1995

(54) **Verfahren zur Herstellung von niedermolekularen Celluloseethern**

Process for preparing low molecular weight cellulose ethers

Procédé pour la préparation d'éthers de cellulose de bas poids moléculaire

(84) Benannte Vertragsstaaten:
BE CH DE FR LI NL

(30) Priorität: 26.09.1994 DE 4434280

(43) Veröffentlichungstag der Anmeldung:
24.04.1996 Patentblatt 1996/17

(73) Patentinhaber: Clariant GmbH
65929 Frankfurt am Main (DE)

(72) Erfinder:
• Dönges, Reinhard, Dr.
D-65812 Bad Soden (DE)
• Reichel, Diethart, Dr.
D-64291 Darmstadt (DE)

(56) Entgegenhaltungen:
DE-A- 1 928 045          DE-A- 3 309 465

**Beschreibung**

Die anwendungstechnischen Eigenschaften von Celluloseethern werden stark von deren Molmasse bzw. der Viskosität ihrer Lösungen beeinflußt. Die Molmasse der Celluloseether ergibt sich dabei aus dem Polymerisationsgrad und der Molmasse der Polymergrundbausteine. Hochmolekulare Celluloseether werden in geringen Konzentrationen als Wasserbindemittel, Verdickungsmittel und Emulsionsstabilisatoren, z.B. in der Medizin, Pharmazie, Kosmetik, Papier-, Textil-, Druck- und Baustoffindustrie eingesetzt. Sollen dagegen die Filmbildungseigenschaften des Celluloseethers ausgenutzt werden, ist es notwendig, konzentrierte Lösungen herzustellen. Dies ist mit niedermolekularen Produkten möglich, die in Wasser keine hohe Viskosität entwickeln.

Niedermolekulare Celluloseether sind grundsätzlich auf zwei verschiedenen Wegen herstellbar. Entweder geht man von niedermolekularer Cellulose aus und verethert diese, oder man führt bei oder nach der Herstellung des Celluloseethers eine Depolymerisation bis auf das gewünschte niedrige Molekulargewicht durch.

Am einfachsten ist die Steuerung des Molekulargewichts des Celluloseethers über den Polymerisationsgrad des eingesetzten Zellstoffs zu erreichen. Da die handelsüblichen Zellstoffe aber ein nach unten begrenztes Molekulargewicht besitzen, ist es in der Praxis in der Regel notwendig, bei der Herstellung sehr niedermolekularer Celluloseether einen Depolymerisationsschritt vorzunehmen, welcher produktionsintegriert erfolgen kann.

Der für die Herstellung von niedermolekularen Celluloseethern erforderliche Molekulargewichtsabbau kann unter Einsatz von energiereicher ionisierender Strahlung wie Elektronenstrahlen oder γ-Strahlen erfolgen.

Die γ-Bestrahlung weist gegenüber der Elektronenbestrahlung mehrere Nachteile auf. Zum einen erfordert eine permanente Strahlungsquelle aufwendige Abschirmungsmaßnahmen, und die Strahlungsquelle emittiert auch bei Nichtbenutzung Strahlung. Zum anderen kommt es bei der Bestrahlung zur Abspaltung von Substituenten vom Celluloseether in Form von Dealkylierungen und Dealkoxylierungen (F.A. Blouin et al., Textile Research Journal 34, 153-158 (1964)).

DE-A-19 28 045 beschreibt ein Verfahren zur Herstellung von wasserlöslichen Celluloseetherprodukten mit niedriger Viskosität durch Elektronenbestrahlung. Als Ausgangsmaterial werden hierbei höhermolekulare und im wesentlichen trockene feste Celluloseetherprodukte eingesetzt, deren Feuchtigkeitsgehalt < 4 Gew.-% ist. Die zu bestrahlende Schicht des in Form von Einzelteilchen vorliegenden Celluloseethers besitzt dabei eine im wesentlichen gleichmäßige Tiefe, welche ungefähr der Eindringtiefe des Elektronenstrahls entspricht. Hieraus resultiert, daß im wesentlichen die gesamte Stahlungsenergie absorbiert wird und eine möglichst gleichmäßige Bestrahlung erfolgt. Die Schicht des zu bestrahlenden Cellulosematerials wird mit einer solchen Geschwindigkeit durch den Elektronenstrahl geschickt, daß die gewünschte Strahlungsdosis erzielt wird. Das auf diese Weise behandelte Produkt wird nach der Bestrahlung gründlich vermischt, um einen Celluloseether mit möglichst gleichmäßigen Eigenschaften zu erhalten.

Es ist ferner bekannt, daß es auch bei der Bestrahlung von festem Cellulosematerial mit Elektronenstrahlung zur Bildung von Carboxylgruppen kommt (F.C. Leavitt, J. Polym. Sci. 51 (1961), 349ff.; K. Fischer, W. Goldberg, M. Wilke, Lenzinger Berichte 59 (1985), 32ff.). Schon nach einer geringen Strahlungsdosis sinkt der pH-Wert einer wäßrigen Lösung des bestrahlten Cellulosematerials durch die gebildeten sauren Gruppen deutlich ab. Eine Erhöhung der Strahlungsdosis führt zu einer weiteren Absenkung des pH-Wertes unter Annäherung an einen Grenzwert, welcher durch den p$K_s$-Wert und die Maximalkonzentration an sauren Gruppen vorgegeben ist. Saure Gruppen beeinträchtigen die Lagerstabilität des Celluloseethers nachhaltig und können zu Vernetzungen führen, wodurch die anwendungstechnischen Eigenschaften erheblich verändert werden.

Wäßrige Lösungen von Celluloseethern, die zuvor in hinreichend trockenem Zustand zum Zweck des Molekulargewichtsabbaus mit ionisierender Strahlung, wie γ-Strahlung oder Elektronenstrahlung, bestrahlt wurden, zeigen bei der Lagerung eine stetig fortschreitende Viskositätsabnahme. Aus US-A-3,108,890 ist bekannt, daß eine derartige Viskositätsabnahme nicht auftritt, wenn der bestrahlte Celluloseether in einem wäßrigen Medium aufgelöst wird und dieser Lösung anschließend eine gewisse Menge einer alkalischen Verbindung zugesetzt wird, die so bemessen ist, daß die Lösung des Celluloseethers einen pH-Wert zwischen 5,5 und 11,5 besitzt. Als alkalische Verbindungen werden Alkalimetall- und Erdalkalimetallhydroxide und Salze schwacher Säuren sowie Ammoniak, Amine und quaternäre Ammonium-Verbindungen oder Komplexe eingesetzt.

Aus US-A-2,895,891 ist ferner bekannt, daß bei der Bestrahlung von Salzen von Cellulosematerialien, z. B. Natrium-Carboxymethylcellulose, mit ionisierender Strahlung ein Molekulargewichtsabbau eintritt, und zwar unabhängig davon, ob die Salze der Cellulosematerialien in wäßriger Lösung oder in trockenem Zustand bestrahlt werden.

Aufgabe der vorliegenden Erfindung ist es, ein Verfahren zur Herstellung von niedermolekularen Celluloseethern zur Verfügung zu stellen, so daß die erhaltenen niedermolekularen Celluloseether eine hohe Lagerstabilität besitzen.

Gegenstand der Erfindung ist ein Verfahren zur Herstellung von niedermolekularen Celluloseethern durch Bestrahlung eines höhermolekularen Celluloseethers mit beschleunigten Elektronen, welches dadurch gekennzeichnet ist, daß eine Mischung aus einem höhermolekularen nichtionischen Celluloseether und einer Base der Elektronenstrahlung ausgesetzt wird.

Als Ausgangsmaterial dienen höhermolekulare nichtionische Celluloseether, die je nach Veretherungsgrad eine mittlere Molmasse $\overline{M}_n$ von 50.000 bis 500.000 g/mol besitzen. Zur Ermittlung der mittleren Molmasse $\overline{M}_n$ der Celluloseether werden Lösungen der Celluloseether mit dem Ubbelohde Kapillarviskosimeter vermessen und dann nach Staudinger das Viskositätsmittel der Molmasse $\overline{M}_n$ errechnet (siehe z.B. Krässig, A., "Cellulose: Structure, accessibility and reactivity, Gordon & Breach Science Publishers, Yverdon, Schweiz, 1993, S.44 ff). Einem Viskositätsmittel der Molmasse $\overline{M}_n$ von 50.000 bis 500.000 g/mol entspricht eine Lösungsviskosität im Bereich von 20 bis über 200.000 mPa·s (gemessen nach DIN 53015 bei 20 °C in einer 2 Gew.-%igen wäßrigen Lösung im Höpplerschen Kugelfallviskosimeter).

Im erfindungsgemäßen Verfahren können alle gängigen nichtionischen Celluloseether mit entsprechend hohem Molekulargewicht eingesetzt werden. Ihre Herstellung erfolgt nach bekannten Verfahren durch Veretherung von Cellulose in alkalischem Medium. Gebräuchliche Veretherungsreagenzien für die Cellulose sind beispielsweise Alkylhalogenide oder Alkylenoxide.

Geeignete Celluloseether sind nichtionische Celluloseether wie Alkylcelluloseether, z.B. Methylcellulose (MC) oder Ethylcellulose (EC), Hydroxyalkylcelluloseether, z.B. Hydroxyethylcellulose (HEC) oder Hydroxypropylcellulose (HPC) oder deren Mischether wie Alkylhydroxyalkylcelluloseether, z.B. Methylhydroxyethylcellulose (MHEC) oder Methylhydroxypropylcellulose (MHPC), Butylhydroxyethylcellulose (BHEC), Ethylhydroxyethylcellulose (EHEC), Methylhydroxybutylcellulose (MHBC), Alkylhydroxyalkylhydroxyalkylcelluloseether, z.B. Methylhydroxyethylhydroxypropylcellulose (MHEHPC).

Bevorzugt werden im erfindungsgemäßen Verfahren Methylcellulose und nicht-ionische Methylcellulosemischether sowie Hydroxyethylcellulose und nichtionische Hydroxyethylcellulosemischether eingesetzt. Unter nichtionischen Methyl- bzw. Hydroxyethylcellulosemischether werden dabei Cellulosematerialien verstanden, die neben den Methyl- bzw. Hydroxyethylsubstituenten nichtionische Alkyl- und/oder Hydroxyalkyl-Substituenten mit 1 bis 4 Kohlenstoffatomen in der Alkylkette enthalten.

Besonders bevorzugt werden im erfindungsgemäßen Verfahren Methylcellulose und nichtionische Methylcellulosemischether wie Methylhydroxyethylcellulose (MHEC), Methylhydroxypropylcellulose (MHPC) sowie Hydroxyethylcellulose und nichtionische Hydroxyethylcellulosemischether wie Hydroxyethylhydroxypropylcellulose (HEHPC) eingesetzt.

Die im erfindungsgemäßen Verfahren einzusetzenden Celluloseether besitzen üblicherweise einen Feuchtigkeitsgehalt von maximal 8 Gew.-%, bezogen auf die Gesamtmasse des Celluloseethers und ein Schüttgewicht von 0,2 bis 0,7 g/cm³. Bei Schüttgütern wird die Dichte in Form des Schüttgewichtes angegeben.

Als basische Verbindungen werden wasserlösliche anorganische Salze eingesetzt, die eine Pufferwirkung besitzen. Bevorzugt werden die Carbonate, Hydrogencarbonate, Phosphate und/oder Hydrogenphosphate der Alkalimetalle eingesetzt, besonders bevorzugt sind die Carbonate und/oder Hydrogencarbonate der Alkalimetalle.

Die der Bestrahlung mit beschleunigten Elektronen auszusetzende Mischung aus höhermolekularem Celluloseether und Base wird üblicherweise hergestellt, indem man zunächst den Celluloseether und die Base getrennt voneinander fein mahlt und absiebt (bei Celluloseethern in Pulverform bis auf eine Korngröße < 200 μm und bei Celluloseethern in Granulatform bis auf eine Korngröße < 500 μm). Im Anschluß an den Siebvorgang wird in einem geeigneten Mischaggregat, beispielsweise einem Pflugscharmischer oder einem Schrägblattmischer, eine innige Mischung aus dem Celluloseether und der basischen Substanz hergestellt.

In einer weiteren Ausführungsform wird der höhermolekulare Celluloseether mit der wäßrigen Lösung einer Base besprüht und anschließend der feuchte Celluloseether zur Entfernung des überschüssigen Wassers und zur gleichmäßigen Einstellung der vorstehend genannten Korngröße einer Mahltrocknung unterworfen.

Im allgemeinen beträgt der Basengehalt 0,01 bis 4 Gew.-%, bezogen auf den Celluloseether. Bevorzugt werden 0,1 bis 2 Gew.-% und besonders bevorzugt 0,1 bis 1 Gew.-% der Base, bezogen auf den Celluloseether, eingesetzt.

Als Strahlenquellen eignen sich sowohl kontinuierliche als auch gepulste Elektronenstrahlbeschleuniger, d. h. dem Celluloseether kann eine bestimmte Strahlungsdosis entweder kontinuierlich mit geringer Intensität oder in kurzen Intervallen mit höherer Intensität zugeführt werden. Bei der Elektronenbestrahlung dringen die beschleunigten Elektronen in die bestrahlte Materie ein, dabei kommt es zur Anregung und Ionisierung von Molekülen im bestrahlten Material (teilweise unter Bildung von Sekundärelektronen) und zum Bruch von Bindungen in der Hauptkette des Makromoleküls unter Bildung von Radikalen, wodurch das mittlere Molekulargewicht reduziert wird.

Zur Charakterisierung des Verfahrens der Elektronenbestrahlung ist besonders die Energiedosis, d.h. die übertragene Strahlungsenergie pro Masseneinheit, von Bedeutung. Üblicherweise beträgt die Energiedosis 5 bis 500 kGy (1 kGy = 1 kJ/kg).

Die Eindringtiefe von hochenergetischen Elektronen ist von ihrer Energie abhängig, sowie von der Dichte des zu bestrahlenden Materials. Eine Berechnung der Eindringtiefe s ist nach folgender Formel empirisch möglich (Formel I)

$$s = 1/\rho \ (5,1 \cdot E-2,6) \tag{I}$$

wobei s die Eindringtiefe in mm, $\rho$ die Dichte (hier: Schüttgewicht) des zu bestrahlenden Materials in $g/cm^3$ und E die Energie des Strahls der beschleunigten Elektronen in MeV ist.

Die Energie des Strahls der beschleunigten Elektronen hängt von der Beschleunigungsspannung ab. Somit ist für ein Material mit einer gegebenen Dichte die nutzbare Eindringtiefe durch die Wahl der Beschleunigungsspannung einstellbar. Das Schüttgewicht der höhermolekularen Celluloseether liegt im allgemeinen zwischen 0,2 und 0,7 $g/cm^3$. Prinzipiell ist es vorteilhaft, mit Beschleunigungsspannungen über 1 MV zu arbeiten. Bevorzugt sind Beschleunigungsspannungen im Bereich von 1 bis 10 MV, besonders bevorzugt von 5 bis 10 MV. Um eine unnötige Erwärmung des Produktes zu vermeiden, empfiehlt es sich, bei hohen gewünschten Gesamtenergiedosen die Bestrahlung in mehreren Durchläufen mit jeweils einem Bruchteil der angestrebten Gesamtdosis durchzuführen.

Bei einer Dichte (Schüttgewicht) der zu bestrahlenden Celluloseether von 0,2 bis 0,7 $g/cm^3$ beträgt die Eindringtiefe s bei einer Beschleunigungsspannung zwischen 1 und 10 MV bis zu ca. 160 mm.

Die Energieabgabe an das Produkt, d.h. die Energiedosis, ist über die Reichweite der Elektronen nicht konstant, wodurch sich auch die vom Produkt absorbierte Strahlungsdosis (Strahlungsenergie pro Masseneinheit) über die Reichweite der Elektronen ändert.

Als optimale Eindringtiefe s(opt) im Hinblick auf eine möglichst gleichmäßige Strahlungsdosis über die Schichtdicke des Produkts bei einseitiger Bestrahlung wird in der Regel ein Wert von ca. $2/3 \cdot s$ bezeichnet, bei dem die absorbierte Strahlungsenergie auf beiden Produktstirnseiten gleich groß ist, d.h. auf der der Strahlungsquelle zugewandten und der der Strahlungsquelle abgewandten Oberfläche des Produkts.

Eine bessere wirtschaftliche Ausnutzung der Strahlungsenergie kann darüberhinaus erreicht werden, wenn das Produkt von beiden Seiten (gleichzeitig oder nacheinander) bestrahlt wird. In diesem Fall liegt die optimale Schichtdicke s(opt) bei einem Wert von $1,6 \cdot s$.

Üblicherweise wird die zu bestrahlende Mischung aus höhermolekularem Celluloseether und Base in Form einer möglichst gleichmäßig tiefen Schicht durch die Strahlung der beschleunigten Elektronen geführt, beispielsweise auf einem Förderband, und zwar mit einer Geschwindigkeit, die zur Erreichung der gewünschten Strahlungsdosis nötig ist. Die zu verwendende Schichtdicke wird dabei gemäß den zuvor genannten Methoden bestimmt.

Durch die Elektronenbestrahlung der Mischung aus höhermolekularem Celluloseether und Base kann der Polymerisationsgrad derart vermindert werden, daß die bestrahlten Celluloseether in einer 2 Gew.-%igen wäßrigen Lösung Viskositäten bis unter 2 mPa·s aufweisen (gemessen nach DIN 53015). Vorzugsweise besitzen die nach dem erfindungsgemäßen Verfahren hergestellten Celluloseether eine Lösungsviskosität von 2 bis 50 mPa·s, was einem Viskositätsmittel der Molmasse $\overline{M}_n$ von ca. 1.000 bis 50.000 entspricht.

Das erfindungsgemäße Verfahren erlaubt es, die Einstellung des angestrebten niedrigen Molekulargewichts gezielt vorzunehmen, ohne daß eine Nachbehandlung nötig ist. Die zur Depolymerisation einzusetzenden Celluloseether können zuvor problemlos durch Veretherung von Cellulose hohen Polymerisationsgrades hergestellt werden, so daß es nur zu geringen Auswaschverlusten und daher auch nur zu einer niedrigen Abwasserbelastung kommt.

Zugleich werden die Nachteile der Elektronenbestrahlung nach dem Stand der Technik vermieden, indem durch Bestrahlung einer Mischung aus höhermolekularem Celluloseether und Base die sich bei der Bestrahlung bildenden sauren Gruppen abgepuffert werden. Es wird somit ein niedermolekularer Celluloseether erhalten, dessen wäßrige Lösung einen pH-Wert im Bereich von 6 bis 8,5 besitzt, woraus eine hohe Lagerstabilität resultiert.

Überraschenderweise wurde außerdem gefunden, daß sich nach der Elektronenbestrahlung der Mischung aus höhermolekularem Celluloseether und Base sowohl die Löslichkeit der Produkte deutlich verbessert als auch die Trübung einer wäßrigen Lösung der Produkte abnimmt (siehe Tabelle 4).

Damit eignen sich die nach dem erfindungsgemäßen Verfahren hergestellten Celluloseether für Anwendungen, bei denen sowohl die Klarlöslichkeit als auch minimale Rückstandswerte wesentlich sind, wie z.B. Kosmetik-Zubereitungen, feste Dosiereinheiten, Zusatz zu keramischen Massen und Polymerisationshilfsmittel. Feste Dosiereinheiten sind z.B. Tabletten, Dragees, und Kapseln. Zugleich wird die Ware durch die Elektronenbestrahlung sterilisiert.

Die Erfindung wird nachfolgend durch Beispiele erläutert.

## Beispiele

Die Alkylsubstitution wird in der Celluloseetherchemie üblicherweise durch den $DS_{Alkyl}$ beschrieben. Der $DS_{Alkyl}$ ist die mittlere Anzahl an substituierten OH-Gruppen pro Anhydroglucoseeinheit.

Die Hydroxyalkylsubstitution wird üblicherweise durch den $MS_{Hydroxyalkyl}$ beschrieben. Der $MS_{Hydroxyalkyl}$ ist die mittlere Anzahl von Molen des Hydroxyalkylierungsreagenzes, die pro mol Anhydroglucoseeinheit etherartig gebunden sind.

Die Viskosität wurde gemäß DIN 53015 nach der Methode von Höppler in einem Kugelfallviskosimeter an einer 2 gew.-%igen wäßrigen Lösung bei 20°C bestimmt.

**Beispiele 1-4**

Bestrahlung von Methylhydroxypropylcelluloseether (MHPC) in Gegenwart von Natriumcarbonat.

1 kg einer Methylhydroxypropylcellulose ($DS_{Methyl}$ = 1,91; $MS_{Hydroxypropyl}$ = 0,19) wurde mit 100 ml einer wäßrigen Natriumcarbonat-Lösung besprüht, deren Konzentration so bemessen war, daß 0,3 bis 0,6 Gew.-% Natriumcarbonat, bezogen auf den Celluloseether, eingesetzt wurden.

Anschließend wurde das Produkt einer Mahltrocknung unterworfen.

In den Beispielen 1 bis 4 erfolgte die Elektronenbestrahlung bei einer Schichtdicke von ca. 6 cm in Einzeldosen von ca. 10 bis 30 kGy und bei einer Beschleunigungsspannung von 10 MV. In Tabelle 1 sind die gemessenen Viskositäten sowie die pH-Werte aufgelistet, wobei Beispiel 1 ein Vergleichsbeispiel darstellt, bei dem der Celluloseether unter den angegebenen Bedingungen, aber ohne Zusatz von Natriumcarbonat bestrahlt wurde.

Tabelle 1:

| Elektronenbestrahlung einer MHPC (DS 1,91; MS 0,19) in Gegenwart von Natriumcarbonat | | | | |
|---|---|---|---|---|
| Beispiel | Gew.-% $Na_2CO_3$ | Gesamtdosis [Gy] | Viskosität (2 Gew.-%ig) [mPa·s] | pH-Wert (2 Gew.-%ig in Wasser) |
| 1 | 0 | 0 | 210 | 5,60 |
| | | 20,3 | 39 | 4,34 |
| | | 50,3 | 19 | 4,06 |
| | | 93,4 | 7,5 | 3,89 |
| | | 163,2 | 4,6 | 3,76 |
| 2 | 0,30 | 0 | 170 | 9,91 |
| | | 50,3 | 16 | 7,18 |
| | | 93,4 | 7,1 | 6,86 |
| | | 163,2 | 4,8 | 6,50 |
| 3 | 0,45 | 0 | 170 | 10,41 |
| | | 50,3 | 14 | 7,99 |
| | | 93,4 | 7,5 | 7,60 |
| | | 163,2 | 4,2 | 7,28 |
| 4 | 0,60 | 0 | 150 | 10,46 |
| | | 93,4 | 7,1 | 7,57 |
| | | 163,2 | 4,2 | 7,30 |

**Beispiele 5 und 6**

Untersuchungen an mit Elektronenstrahlen bestrahlter Methylhydroxypropylcellulose im Hinblick auf Löslichkeit und Trübungsverhalten in wäßriger Lösung.

In Beispiel 6 wurde 1 kg einer Methylhydroxypropylcellulose ($DS_{Methyl}$ 1,81; $MS_{Hydroxypropyl}$ 0,24) mit 100 ml einer wäßrigen Natriumcarbonat-Lösung besprüht, deren Konzentration so bemessen war, daß 0,15 bis 0,6 Gew.-% Natriumcarbonat bezogen auf den Celluloseether, eingesetzt wurden.

Anschließend wurde das Produkt einer Mahltrocknung unterworfen.

Die Elektronenbestrahlung erfolgte bei einer Schichtdicke von ca. 6 cm in Einzeldosen von ca. 20 kGy und bei einer Beschleunigungsspannung von 10 MV.

Beispiel 5 ist ein Vergleichsbeispiel, bei dem der Cellulosemischether unter den angegebenen Bedingungen, aber ohne Zusatz von Natriumcarbonat bestrahlt wurde.

Nach der Elektronenbestrahlung wurde eine 1 Gew.-%ige Lösung der bestrahlten Produkte in Wasser hergestellt, die erhaltene Lösung durch einen Filter mit einer Porengröße 20 µm filtriert und der unlösliche Rückstand bestimmt (Tabelle 2).

Das Trübungsverhalten der bestrahlten Proben wurde ebenfalls an einer 1 gew.-%igen wäßrigen Lösung bei einer Wellenlänge von 578 nm bestimmt, wobei die untersuchte Schichtdicke 1 cm betrug.

Die gefundenen Extinktionswerte sind in Tabelle 2 aufgelistet.

Tabelle 2:

| MHPC nach Elektronenbestrahlung (Strahlungsdosis = 160 kGy) (DS 1,81; MS 0,24) | | | |
|---|---|---|---|
| Beispiel | Gew.-% $Na_2CO_3$ | Rückstand (%) | Extinktion |
| 5 | 0 | 0,031 | 0,046 |
| 6 | 0,15 | 0,016 | 0,021 |
| | 0,3 | 0,018 | 0,022 |
| | 0,45 | 0,019 | 0,017 |
| | 0,6 | 0,018 | 0,019 |

**Beispiel 7**

Herstellung von Filmtabletten

Zur Herstellung einer Beschichtungslösung für Filmtabletten wurde Methylhydroxypropylcellulose (MHPC) ($DS_{Methyl}$ 1,8; $MS_{Hydroxyalkyl} = 0,24$; Viskosität = 4,8 mPa·s) eingesetzt.

| 1 kg Beschichtungslösung bestand aus: | | |
|---|---|---|
| | 80 g | MHPC |
| | 3,5 g | Polyethylenoxid 8000 |
| | 20 g | Titandioxid |
| | 6 g | Talkum |
| | 1 g | Eisenoxid gelb |
| 889,5 g | Wasser | |

Auf Placebo-Tabletten wurde ein 0,1 mm starker Film aufgebracht, der glatt und gleichmäßig war. Der Film führte zu keiner wesentlichen Zerfallsverzögerung der Tabletten.

Der eingesetzte Celluloseether wurde hergestellt durch Abmischen von 1 kg MHPC (Viskosität = 200 mPa·s) mit einer Lösung von 6 g Soda in 100 g Wasser und anschließender Mahltrocknung. Die Elektronenbestrahlung mit 161 kGy lieferte ein Produkt, dessen 2 Gew.-%ige wäßrige Lösung einen pH-Wert von 6,9 aufwies.

**Patentansprüche**

1.  Verfahren zur Herstellung von niedermolekularen Celluloseethern durch Bestrahlung einer Mischung aus einem höhermolekularen nichtionischen Celluloseether und einer Base mit beschleunigten Elektronen.

2.  Verfahren nach Anspruch 1, dadurch gekennzeichnet, daß der höhermolekulare Celluloseether eine mittlere Molmasse $\overline{M}_n$ von 50.000 bis 500.000 g/mol besitzt.

3.  Verfahren nach Anspruch 1, dadurch gekennzeichnet, daß ein Methylcelluloseether oder ein nichtionischer Methylcellulosemischether eingesetzt wird.

4.  Verfahren nach Anspruch 1, dadurch gekennzeichnet, daß ein Hydroxyalkylcelluloseether oder ein nichtionischer Hydroxyalkylcellulosemischether eingesetzt wird.

5.  Verfahren nach Anspruch 1, dadurch gekennzeichnet, daß als Base ein wasserlösliches anorganisches Salz eingesetzt wird, das eine Pufferwirkung besitzt.

6.  Verfahren nach Anspruch 1, dadurch gekennzeichnet, daß der Basengehalt 0,01 bis 4 Gew.-%, bevorzugt 0,1 bis 2 Gew.-%, besonders bevorzugt 0,1 bis 1 Gew.-%, bezogen auf den Celluloseether, beträgt.

**7.** Verfahren nach Anspruch 1, dadurch gekennzeichnet, daß die Mischung aus dem nichtionischen Celluloseether und der Base hergestellt wird, indem zunächst der Celluloseether und die Base getrennt voneinander fein gemahlen werden und anschließend in einem Mischaggregat gemischt werden.

**8.** Verfahren nach Anspruch 1, dadurch gekennzeichnet, daß der Celluloseether mit der wäßrigen Lösung der Base besprüht wird und anschließend der feuchte Celluloseether einer Mahltrocknung unterworfen wird.

**9.** Verfahren nach Anspruch 1, dadurch gekennzeichnet, daß das Schüttgewicht des zu bestrahlenden Celluloseethers 0,2 bis 0,7 $g/cm^3$ beträgt.

**10.** Verfahren nach Anspruch 1, dadurch gekennzeichnet, daß die Bestrahlung mit Hilfe eines kontinuierlichen Elektronenstrahlbeschleunigers erfolgt.

**11.** Verfahren nach Anspruch 1, dadurch gekennzeichnet, daß die Energiedosis der Elektronenstrahlung 5 bis 500 kGy (kJ/kg) beträgt.

**12.** Verwendung der nach dem Verfahren gemäß Anspruch 1 hergestellten niedermolekularen Celluloseether als Überzugsmaterial für feste Dosiereinheiten.

**13.** Verwendung der nach dem Verfahren gemäß Anspruch 1 hergestellten niedermolekularen Celluloseether als Zusatz in keramischen Massen, Kosmetik-Zubereitungen oder als Polymerisationshilfsmittel.

**Claims**

**1.** A process for the preparation of a low molecular weight cellulose ether by irradiation of a mixture of a higher molecular weight nonionic cellulose ether and a base with accelerated electrons.

**2.** The process as claimed in claim 1, wherein the higher molecular weight cellulose ether has an average molecular weight $\overline{M}_\eta$ of 50,000 to 500,000 g/mol.

**3.** The process as claimed in claim 1, wherein a methylcellulose ether or a nonionic methylcellulose mixed ether is employed.

**4.** The process as claimed in claim 1, wherein a hydroxyalkylcellulose ether or a nonionic hydroxyalkylcellulose mixed ether is employed.

**5.** The process as claimed in claim 1, wherein a watersoluble inorganic salt which has a buffer action is employed as the base.

**6.** The process as claimed in claim 1, wherein the base content is 0.01 to 4 % by weight, preferably 0.1 to 2 % by weight, particularly preferably 0.1 to 1 % by weight, based on the cellulose ether.

**7.** The process as claimed in claim 1, wherein the mixture of the nonionic cellulose ether and the base is prepared by first finely grinding the cellulose ether and the base separately from one another and then mixing them in a mixing unit.

**8.** The process as claimed in claim 1, wherein the cellulose ether is sprayed with the aqueous solution of the base and the moist cellulose ether is then subjected to drying by grinding.

**9.** The process as claimed in claim 1, wherein the bulk density of the cellulose ether to be irradiated is 0.2 to 0.7 $g/cm^3$.

**10.** The process as claimed in claim 1, wherein the irradiation is carried out with the aid of a continuous electron beam accelerator.

**11.** The process as claimed in claim 1, wherein the energy dose of the electron beam is 5 to 500 kGy (kJ/kg).

**12.** The use of a low molecular weight cellulose ether prepared by the process as claimed in claim 1 as a coating material for solid dosage units.

13. The use of a low molecular weight cellulose ether prepared by the process as claimed in claim 1 as an additive in ceramic compositions or cosmetics formulations or as a polymerization auxiliary.

**Revendications**

1. Procédé pour la préparation d'éthers cellulosiques de bas poids moléculaire par irradiation d'un mélange constitué d'un éther cellulosique de haut poids moléculaire non ionique et d'une base, par des électrons accélérés.

2. Procédé selon la revendication 1, caractérisé en ce que l'éther de haut poids moléculaire présente une masse molaire moyenne $\overline{M}_n$ de 50 000 à 500 000 g/mole.

3. Procédé selon la revendication 1, caractérisé en ce que l'on utilise un éther de méthylcellulose ou un éther mixte de méthylcellulose non ionique.

4. Procédé selon la revendication 1, caractérisé en ce que l'on utilise un éther hydroxyalkylcellulosique ou un éther mixte hydroxyalkylcellulosique non ionique.

5. Procédé selon la revendication 1, caractérisé en ce que l'on utilise comme base un sel inorganique hydrosoluble, possédant un effet tampon.

6. Procédé selon la revendication 1, caractérisé en ce que la teneur en base est de 0,01 à 4 % en poids, de préférence de 0,1 à 2 % en poids, de manière particulièrement préférée de 0,1 à 1 % en poids, par rapport à l'éther cellulosique.

7. Procédé selon la revendication 1, caractérisé en ce que l'on prépare le mélange de l'éther cellulosique non ionique et de la base, d'abord par broyage fin séparément l'éther cellulosique et la base et ensuite, par mélange dans un appareil de mélange.

8. Procédé selon la revendication 1, caractérisé en ce que l'on arrose l'éther cellulosique avec la solution aqueuse de la base et ensuite on traite l'éther cellulosique humide par broyage à sec.

9. Procédé selon la revendication 1, caractérisé en ce que le poids en vrac de l'éther cellulosique à irradier est de 0,2 à 0,7 g/cm$^3$.

10. Procédé selon la revendication 1, caractérisé en ce que l'on réalise l'irradiation à l'aide d'un accélérateur de faisceaux d'électrons en continu.

11. Procédé selon la revendication 1, caractérisé en ce que la dose d'énergie du rayonnement électronique est de 5 à 500 kGy (kJ/kg).

12. Utilisation de l'éther cellulosique de bas poids moléculaire préparé selon le procédé de la revendication 1, en tant que matière de revêtement pour des unités-doses solides.

13. Utilisation de l'éther cellulosique de bas poids moléculaire préparé selon le procédé de la revendication 1, en tant qu'additif dans des matières céramiques, des préparations cosmétiques ou comme adjuvant de polymérisation.